# EUROPEAN PATENT SPECIFICATION

(11) **EP 1 231 874 B1**
(45) Date of publication and mention of the grant of the patent: **02.03.2005**
(21) Application number: 00928033.0
(22) Date of filing: 17.04.2000
(51) Int. Cl.: A61F 5/02

(54) **ADJUSTABLE SPINAL BRACE**
ANPASSBARE WIRBELSÄULENSTÜTZE
CORSET ORTHOPEDIQUE REGLABLE

(30) Priority: 30.04.1999 SE 9901556
(43) Date of publication of application: 21.08.2002
(73) Proprietor: CAMP SCANDINAVIA AB, 254 67 Helsingborg (SE)
(72) Inventor: WILLNER, Stig DI, - (NL); SMITS, Jan, F., A., NL-5709 MP Helmond (NL)
(74) Representative: Akerman, Marten Lennart
(86) International application number: PCT/SE2000/000727
(87) International publication number: WO 2000/066047

(56) References cited:
- DE-A1- 3 522 533
- SE-B- 454 944
- US-A- 2 434 883
- US-A- 3 945 376
- US-A- 4 821 739

## Description

The present invention relates to an adjustable spinal brace, according to the preamble of claim 1.

### State of the art

Various braces and other orthotic devices are known in the art. There are spinal braces with various heights, some intending to immobilise and stabilise the entire spine - thoracic as well as lumbar areas. Other types of spinal braces - low type - are only stabilising the lower part of the spine - the lumbar area.

Furthermore, the rigidity of the brace varies a lot from soft braces made of fabrics to rigid braces made of plastics. The more rigid the brace is, the more immobilising and stabilising effects are seen. That means that a rigid brace, nowadays usually prefabricated by thermoplastic materials, has a better positive pain relieving effect compared to soft and semirigid braces.

However, because of the rigidity of the brace, a lot of negative effects are seen such as an irritating stiffness of the trunk and unpleasant diffuse pressure on the trunk. Furthermore, the pressure on the groins will negatively influence the mobility of the hips seen as difficulties to drive cars etc. Other negative effects are excessive pressure of the upper border of the rigid brace on the thoracic cage, and the rigidity also makes movements of the arms more difficult.

Not very seldom, the negative effects will exceed the positive effects. The consequence of this is that, in spite of a clear pain relief when using the brace, the patients will not accept and use the brace.

It is known that the position of the spine giving a maximal pain relief is difficult to find. Even just small changes of the degree of the lumbar lordosis and the level of this maximal support will influence the effect of the brace treatment. This position for a maximal pain relief varies from one case to another, which means that a prefabricated shaped brace will not give the patient the maximal pain relief.

Typically, prior art braces comprise frontal breast or abdominal plates or elements and anterior dorsal plates or elements interconnected by straps and rolls to apply stabilising or immobilising pressures. Examples of the prior art may be found in the US patents Nos. 2,813,526, 4,285,336 and 4,930,499. A problem with the prior art is that it is difficult to set the exact magnitude and position of the pressure by means of tightening straps. In this connection, it is critical that the position and magnitude of the pressure applied is correct because otherwise the patient does not experience any pain relief.

As is mentioned above, another problem is that the abdominal and dorsal elements are large and encumber the movements of the body, e.g. lateral bending and movements of the arms and legs as well as sitting.

In this technical field, a test instrument for testing spinal braces is previously known. The test instrument was developed by one of the present inventors and patented in Sweden under No. 8505547-3, corresponding to US 4 821 739. The test instrument includes various means for adjusting the size of the brace and magnitude and position of the applied pressure. However, this test instrument is not suitable for day to day use because the design featured protruding screws and the like and was not generally designed for wearing under clothes. Even so, the test instrument was sometimes used as a brace for shorter periods.

US Patent No.3,945,376 describes a spinal orthosis provided with iliac elements (iliac crests). The preamble of claim 1 is based on this document. The iliac crests are formed of a flexible material, such as a rubber hose. A pelvic band of the orthosis is placed round the hip and not connected to the iliac crests. This results in that it is difficult to put on or take of the orthosis, especially to place it reproducibly in the correct position. Moreover, the orthosis lacks a lumbar pad to adjust the shape of the lordosis. The orthosis is just applied with varying pressure in the different parts.

The present invention solves the above problems by providing an adjustable spinal brace which is easily and reproducibly positioned on the body of a patient thanks to a connection means having lateral iliac rolls which are fitted at the top of the pelvis bone. Also, it is easy to adjust the magnitude and position of the applied pressure thanks to a lumbar pad provided with pressure setting means. Also, the lumbar pad may be positioned at different heights and the pressure point may be shifted in a lateral direction.

### Summary of the invention

The invention is defined in the characterising portion of claim 1 while preferred embodiments are set forth in the dependent claims.

### Brief description of the drawings

The invention will be described in detail below with reference to the accompanying drawings, in which:
figure 1 is a dorsal perspective view of the spinal brace according to the invention,
figure 2 is a front view of the brace as worn on a patient with the skeleton bones visible,
figure 3 is a side view similar to figure 2,
figure 4 is a partially cut-away dorsal view of a screw jack design according to the invention,
figure 5A is a side cut-away view of the screw jack in a retracted position,
figure 5B is a side view of the screw jack in an extended position, and
figure 6 is a top detail view of the connection between the posterior and anterior parts.

### Detailed description of preferred embodiments

The invention provides an adjustable spinal brace, especially a lumbar brace, which is multi-adjustable for conservative treatment of low back pain. The pain may have various causes, such as discogenic hernia, spinal stenose, spondylotisthesis etc. The brace of the invention is a further development of the test instrument mentioned in the introduction, which is a tool to prove that conservative treatment could be effective. The present brace is designed for individual use during day and night. The design of the brace if virtually open at the lateral side, which allows for lateral bending. In general, the brace stabilises the spine in the sagittal plane.

One purpose of the brace is to stabilise the lumbar spine by the use of a "three point pressure" brace. Also, the brace restores lumbar lordosis to the individual needs by a multi-adjustable posterior lumbar pad.

A lightweight posterior frame bridges the lumbar spine from the distal end of the sacrum until the tenth thoracic vertebra T10, approximately two fingers below the scapulae. Two iliac rolls, connected to the posterior frame, position this frame over the pelvis. In this situation the frame finds a reliable "grip" between the lower ribs and the pelvis, which can be maintained efficiently. The iliac rolls have virtually just "one" position where they can actually be. This is extremely important, since the adjustments of the posterior lumbar pad 2 and the accuracy of this position after putting on the brace relies fully on the position of the iliac rolls, which at the same time will pull the abdominal pad into the soft tissue of the abdomen to create the third point in achieving the desired stabilisation of the lumbar spine. When properly applied to the body, the "three point pressure" will stabilise the lumbar spine into a more or less lordotic position with reduced lordosis. All functional parts are integrated within the frame and do hardly interfere with activities like sitting, standing, walking or lying.

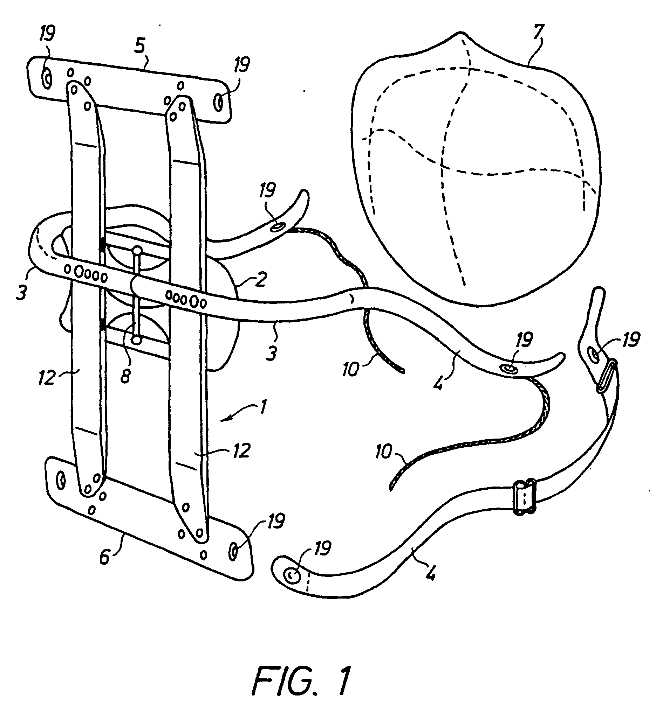

Figure 1 shows the brace according to the invention in a partially exploded view. The brace has a posterior frame 1 to be placed at the back of the patient. The frame 1 comprises two horizontal supports, a cranial support 5 and a caudal support 6. The horizontal supports 5, 6 are attached to two vertical H-shaped struts 12 by means of a respective set of screws.

The horizontal cranial support 5 can be adjusted vertically over a range of 50 mm. Also the caudal support 6 can be adjusted vertically over a range of 50 mm. The total height of the frame I can be adjusted from 390 to 490 mm. This adjustment range is sufficient for 98 % of the population. The dimensions may of course be varied to provide other sizes.

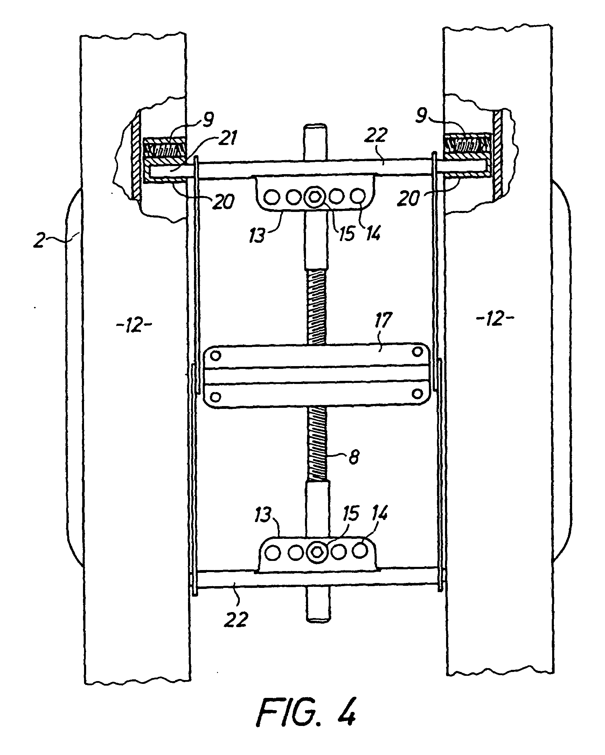

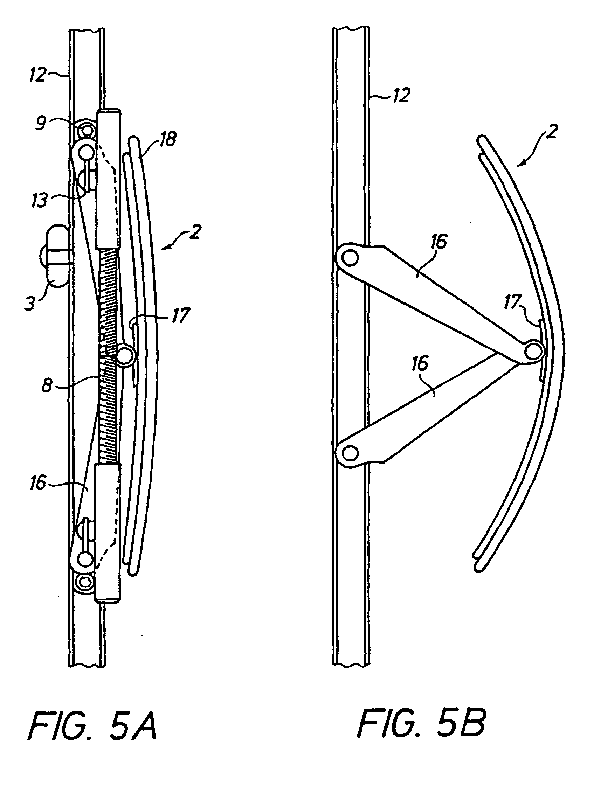

In the centre of the posterior frame 1 two iliac rolls 3 are provided. The iliac rolls 3 are intended to be positioned at the crista iliaca, that is they will rest on top of the pelvis bone. The iliac rolls are attached by means of elastic straps 4 and push buttons 19 to an anterior abdominal pad 7. Also the cranial and caudal supports 5, 6 may be connected with straps to the abdominal pad 7. Only one lower strap 4 is shown in figure 1. The posterior lumbar pad 2 is attached to the posterior frame 1 as is described more in detail with reference to figures 4, 5A and 5B.

Seen in a horizontal section, the vertical struts 12 consist of an "H" profile, with lateral openings. The shape of the struts 12 along the back of the patient is adapted to the anatomical shape of the human body. The openings and flanges of the "H" profile are useful for attachment of the horizontal supports 5, 6, the iliac rolls as well as a multi-adjustable posterior lumbar pad 2. For comfort the cranial and caudal horizontal supports are padded with foam (not shown).

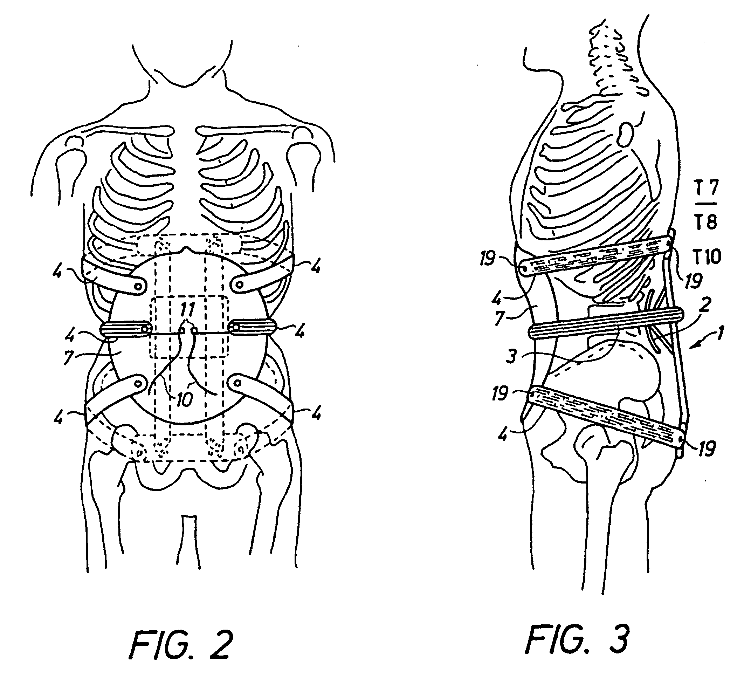

In figure 2, the brace is shown from the front as fastened on a patient. The skeleton bones on the patient are indicated for reference. As may be seen the iliac rolls and their straps 4 are located at the waist.

Figure 3 shows a similar view to figure 2 from the side. As is more clearly seen in figure 3, the iliac rolls 3 are resting against crista iliaca (with some soft tissue in between).

Figure 4 is a dorsal view of the suspension mechanism for the posterior lumbar pad 2. See also figures 5A and 5B. The lumbar pad 2 is attached by means of a mounting plate 17. The mounting plate 17 has a shaft such that the pad 2 may be rotated round a horizontal axis. The mounting plate 17 is connected by two pairs of legs 16 to the vertical struts 12. The legs 16 have pins 21 at the ends remote from the mounting plate 17. The pins are inserted in blocks 20 sliding in the H-shaped struts 12. Only the top blocks 20 are shown in figure 4. The blocks 20 may also carry set screws 9 for securing the lumbar pad 12 at a suitable height as is described more in detail below.

The pins 21 are carried by or integral with a respective sleeve 22. The sleeve 22 also carries a bracket 13 with a row of holes 14. An adjustment screw 8 is fastened in one pair of the holes 14, normally the centre pair. As is shown, the screw 8 has a pair of oppositely directed threads, such that the distance between the top and lower sleeve 22 may be adjusted accurately by screwing the screw 8. The screw 8 may be shifted to the left and right by using another pair of holes 14 for the reason described more in detail below.

Figure 5A shows the lumbar pad 2 in its completely retracted position. In this position the pad 2 is hardly touching the back of the patient but hidden in within the posterior frame 1. In figure 5B the pad is shown in an extended position. The maximum extension may be e.g. 50 mm.

The iliac rolls 3 are connected via elastic straps 4 with push buttons 19, strings 10 and string locks 11 to the anterior abdominal pad 7 for applying the abdominal pressure.

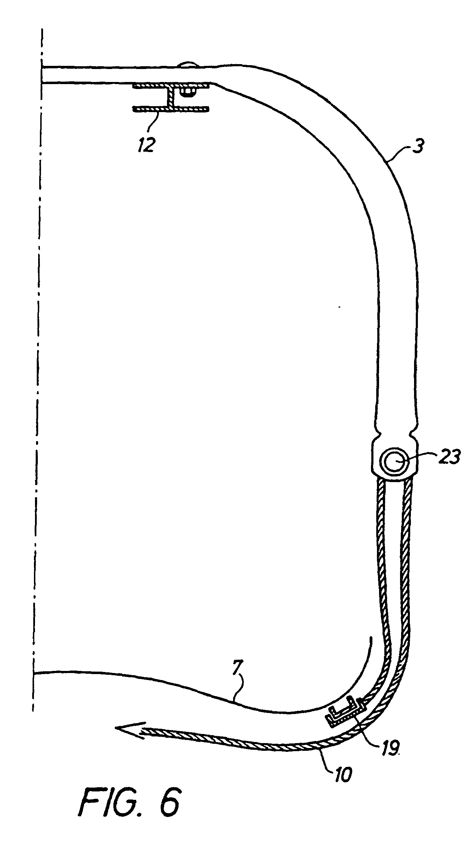

In the embodiment shown in figure 6, the strings 10 may be guided around a respective pulley block 23. One end of the string is attached to the abdominal pad at the push button (via the strap 4) and the other end of the string is attached to the string lock 11 after tightening. The string lock 11 and pulley block 23 make it possible to apply sufficient pressure in an efficient and easy way by tightening the strings 10 attached to the elastic straps 4 of the iliac rolls 3. This arrangement is easy to handle for the patient or doctor to achieve a sufficient tension and pressure.

Figure 6 also shows how the iliac rolls are attached to the H-shaped struts with screws. As may be seen from figure 1, the iliac rolls carry a number of holes so that the width of the brace is easily adjusted by using the set of holes providing the suitable width for the patient.

The anterior abdominal pad 7 applies the abdominal lumbar pressure. It is a lightweight concave pad, which should cover the abdomen from the symphysis up to the sternum point. From left lateral to right lateral the abdominal pad 7 should bridge the distance between the two iliac rolls 3. The cranial border of the pad is shaped after the lower ribs. The abdominal pad should be able to apply considerable pressure without changing shape and is therefore constructed out of a thermosetting composite. The pad may be tailored individually for the patient. The pad is furnished with (parts of) pushbuttons for attachment of the straps 4 and string locks 11 for the strings. For comfort the pad is lined with foam padding (not shown).

Depending on the indication, the lordosis could be restored to the individual needs by means of the multi-adjustable posterior lumbar pad 2. In a non-functional position this lumbar pad will be hidden in the frame. Then, the three point pressure is applied by the horizontal support 5 and the horizontal caudal support 6 together with the anterior abdominal pad 7.

The pad can be moved vertically by sliding in the H-shaped vertical struts 12. The pad itself is curved and somewhat flexible to follow the curvature of the lumbar portion of the back. For comfort the pad is lined with foam padding 18. It is suspended by means of a mounting plate 17 and a shaft such that it can turn around a horizontal axis. The centre can be positioned from vertebra L4 through T12; the most common area will be from L3 to L1. By means of the vertically placed adjusting screw 8, preferably a socket head cap screw, the posterior lumbar pad can be adjusted to apply pressure in the anterior direction. The lumbar pad 2 is maintained at the suitable height by means of the pressure and friction against the back of the patient. As an alternative, set screws 9 can keep the lumbar pad in place.

If the reaction force from the spine and soft tissue differs between left and right the mechanism will respond asymmetrically (like a trapezoid with two parallel vertical sides). If for instance the right vertical side is longer, the pressure at the right side appears to be higher. By shifting the vertical adjusting screw to the right a compensation for this higher pressure can be accomplished. This way it becomes possible to balance the rotational forces of the spine.

A person with low back pain is in general fully capable in deciding in what level (height) and how strong (depth) the pad should be applied. Normally a good starting position is to put the centre of the pad at the level of the L3 vertebra, which is at the 0-level (spine tangent to vertical)of the lordosis (without the compensation provided by the invention). The brace is put on by placing the iliac rolls at the crista iliaca and fastening the straps 4 by means of the push buttons. The straps are tightened by means of the strings 10 and the pulley blocks 23 and the string locks 11 such that a suitable pressure is obtained. If an enhanced stability against side bending is required, also the upper and lower straps 4 are attached to the cranial 5 and caudal supports, respectively. With the help of the screw-jack and a hexagon driver the doctor can apply pressure to the lumbar spine by means of the lumbar pad and restore lordosis against the pressure of the abdominal pad 7. Once the position of the lumbar pad is set correctly, the patient may himself or herself doff and don the brace without requiring help.

The surface area of the anterior abdominal pad needs to be considerable bigger than the surface of the posterior lumbar pad. During forward bending the horizontal cranial 5 and caudal 6 support move away from the body and there is a risk that the pressure of the dorsal pad becomes too high in relation to the pressure of the abdominal pad. The adjustments of the screw-jack can be used to compensate for the counter-pressure of the spine and the soft tissue. Asymmetrical pressure on the posterior lumbar pad will result in an asymmetrical position of the screw-jack. If a compensation of these forces is required the adjusting screw can be shifted to the side where the pressure is highest, thus compensating and restoring the balance. Since the abdominal pressure acts as a counter pressure for all forces the user of the brace can feel these differences at his abdomen as a result of the higher pressure against the abdominal pad.

The following advantages of the invention may be mentioned:
- allowing an individual assessment of the shape of the lumbar lordosis giving a maximal pain relief;
- allowing a support in the optimal pain relieving level of the lumbar spine;
- allowing a sufficient abdominal counterpressure to stabilize the device and keep the correct level of the lumbar lordosis, also to increase the intraabdominal pressure to decrease the intradiscal pressure (pain relieving effect);
- allowing the iliac rolls to be stabilised over the crista iliaca, thus stabilising the device and preventing it from sliding up or down, which would move the dorsal plate up and down and thus loosing the level of optimal pain relief;
- allowing a moderate side-bending of the trunk to improve the acceptance of the brace treatment;
- allowing at least 90° bending of the hip joints i.e. when sitting in a car;
- allowing an improved movement of the arms and the upper part of the trunk and avoiding unpleasant pressure of the upper edges of the device usually seen in conventional rigid braces.

A preferred embodiment of the invention has been described in detail above. Many modifications as to the specific shape and materials used will be readily apparent to a person skilled in the art. The scope of the invention is only limited by the claims below.

## Claims

1. An adjustable spinal brace comprising an anterior abdominal pad (7), a posterior frame (1) and lateral iliac rolls (3), said iliac rolls (3) to be positioned at the top of the pelvis bone (crista iliaca), and the abdominal pad (7) and the posterior frame (1) being interconnected by a connection means, wherein the connection means comprises said iliac rolls (3) and straps (4) to be connected to the anterior abdominal pad (7), **characterised in that** the posterior frame (1) has an adjustable lumbar pad (2) provided with pressure setting means (8), the pressure setting means comprising a screw jack having legs (16) and an adjusting screw (8) connected to said legs (16) for adjusting the distance between the legs and setting the distance between the lumbar pad (2) and the posterior frame (1), thereby enabling an adjustment of the applied pressure.

2. A spinal brace according to claim 1, **characterised in that** the adjusting screw (8) is placed vertically.

3. A spinal brace according to claim 2, **characterised in that** the position of the screw (8) of the screw jack is adjustable in the lateral direction.

4. A spinal brace according to claim 1, 2 or 3, **characterised in that** the lumbar pad also (2) is adjustable in height.

5. A spinal brace according to any of the previous claims, **characterised in that** the posterior frame (1) comprises two vertical struts (12), a cranial horizontal support (5) and a caudal horizontal support (6).

6. A spinal brace according to claim 5, **characterised in that** the cranial horizontal support (5) and the caudal horizontal supports (6) are adjustable in height.

7. A spinal brace according to any of the previous claims, **characterised in that** straps (4) are provided between the iliac rolls (3) and the abdominal pad (7).

8. A spinal brace according to claim 7 and 5 or 6, **characterised in that** straps (4) are provided between the horizontal supports (5, 6) and the abdominal pad (7).

9. A spinal brace according to any of the previous claims, **characterised in that** the anterior abdominal pad (7) is lightweight and concave and adapted to cover the abdomen substantially from the symphysis to the sternum point.

10. A spinal brace according to claim 9, **characterised in that** the cranial border of the anterior abdominal pad (7) is shaped after the lower ribs.

## Patentansprüche

1. Einstellbare Wirbelsäulenstütze, welche ein vorderes Bauchkissen (7), ein hinteres Gestell (1) und seitliche Darmbeinwickel (3) umfasst, wobei die Darmbeinwickel (3) an dem oberen Ende des Beckenknochens (Crista iliaca) zu positionieren sind und das Bauchkissen (7) und das hintere Gestell (1) durch ein Verbindungsmittel miteinander verbunden sind, wobei das Verbindungsmittel die Darmbeinwickel (3) und Riemen (4) umfasst, welche an dem vorderen Bauchkissen (7) zu befestigen sind, **dadurch gekennzeichnet, dass** das hintere Gestell (1) ein einstellbares Lendenkissen (2) aufweist, welches mit Druck-Einstellmitteln (8) ausgestattet ist, wobei die Druck-Einstellmittel eine Schraubenspindel umfassen, welche Stützen (16) und eine Einstellschraube (8) umfasst, wobei Letztere mit den Stützen (16) verbunden ist, zum Einstellen des Abstandes zwischen den Stützen und zum Einstellen des Abstandes zwischen dem Lendenkissen (2) und dem hinteren Gestell (1), um auf diese Weise ein Einstellen des aufgewandten Drucks zu ermöglichen.

2. Wirbelsäulenstütze nach Anspruch 1, **dadurch gekennzeichnet, dass** die Einstellschraube (8) vertikal angebracht ist.

3. Wirbelsäulenstütze nach Anspruch 2, **dadurch gekennzeichnet, dass** die Position der Schraube (8) der Schraubenspindel in der lateralen Richtung regulierbar ist.

4. Wirbelsäulenstütze nach Anspruch 1, 2 oder 3, **dadurch gekennzeichnet, dass** das Lendenkissen (2) auch in der Höhe regulierbar ist.

5. Wirbelsäulenstütze nach einem der vorhergehenden Ansprüche, **dadurch gekennzeichnet, dass** das hintere Gestell (1) zwei vertikale Verstrebungen (12), eine kraniale horizontale Stütze (5) und eine kaudale horizontale Stütze (6) umfasst.

6. Wirbelsäulenstütze nach Anspruch 5, **dadurch gekennzeichnet, dass** die kraniale horizontale Stütze (5) und die kaudale horizontale Stütze (6) in der Höhe regulierbar sind.

7. Wirbelsäulenstütze nach einem der vorhergehenden Ansprüche, **dadurch gekennzeichnet, dass** die Riemen (4) zwischen den Darmbeinwickeln (3) und dem Bauchkissen (7) vorgesehen sind.

8. Wirbelsäulenstütze nach Anspruch 7 und 5 oder 6, **dadurch gekennzeichnet, dass** die Riemen (4) zwischen den horizontalen Stützen (5, 6) und dem Bauchkissen (7) vorgesehen sind.

9. Wirbelsäulenstütze nach einem der vorhergehenden Ansprüche, **dadurch gekennzeichnet, dass** das vordere Bauchkissen (7) einen leichten und konkaven Zustand aufweist und angepasst ist, um den Bauch im Wesentlichen von der Symphyse bis zum Sternum abzudecken.

10. Wirbelsäulenstütze nach Anspruch 9, **dadurch gekennzeichnet, dass** der kraniale Rand des vorderen Bauchkissens (7) nach den unteren Rippen geformt ist.

## Revendications

1. Corset lombaire réglable comprenant un coussin abdominal antérieur (7) et un châssis postérieur (1) et des rouleaux iliaques latéraux (3), lesdits rouleaux iliaques (3) devant être positionnés en haut de l'os pelvien (crête iliaque), et le coussin abdominal (7) et le châssis postérieur (1) étant interconnectés par des moyens de raccordement, dans lequel les moyens de raccordement comprennent lesdits rouleaux iliaques (3) et des bandes (4) devant être connectées au coussin abdominal antérieur (7), **caractérisé en ce que** le châssis postérieur (1) a un coussin lombaire réglable (2) pourvu de moyens de réglage de pression (8), les moyens de réglage de pression (8) comprenant un vérin à vis ayant des jambes (16) et une vis de réglage (8) raccordée auxdites jambes (16) pour ajuster la distance entre les jambes et régler la distance entre le coussin lombaire (2) et le châssis postérieur (1), permettant ainsi un réglage de la pression appliquée.

2. Corset lombaire selon la revendication 1, **caractérisé en ce que** la vis de réglage (8) est placée verticalement.

3. Corset lombaire selon la revendication 2, **caractérisé en ce que** la position de la vis (8) du vérin à vis est réglable dans la direction latérale.

4. Corset lombaire selon la revendication 1, 2 ou 3, **caractérisé en ce que** le coussin lombaire (2) est également réglable en hauteur.

5. Corset lombaire selon l'une quelconque des revendications précédentes, **caractérisé en ce que** le châssis postérieur (1) comprend deux entretoises verticales (12), un support horizontal crânien (5) et un support horizontal caudal (6).

6. Corset lombaire selon la revendication 5, **caractérisé en ce que** le support horizontal crânien (5) et le support horizontal caudal (6) sont réglables en hauteur.

7. Corset lombaire selon l'une quelconque des revendications précédentes, **caractérisé en ce que** les bandes (4) sont prévues entre les rouleaux iliaques (3) et le coussin abdominal (7).

8. Corset lombaire selon la revendication 7 et 5 ou 6, **caractérisé en ce que** les bandes (4) sont prévues entre les supports horizontaux (5, 6) et le coussin abdominal (7).

9. Corset lombaire selon l'une quelconque des revendications précédentes, **caractérisé en ce que** le coussin abdominal antérieur (7) est léger et concave et adapté pour couvrir l'abdomen essentiellement à partir des symphyses jusqu'au point du sternum.

10. Corset lombaire selon la revendication 9, **caractérisé en ce que** la frontière crânienne du coussin abdominal antérieur (7) est formée après les côtes inférieures.
